# EUROPEAN PATENT SPECIFICATION

(11) **EP 0 546 076 B1**
(45) Date of publication and mention of the grant of the patent: **20.05.1998**
(21) Application number: 91916518.3
(22) Date of filing: 30.08.1991
(51) Int. Cl.: A61K 35/14, A61K 39/395

(54) **Use of anti-ICAM antibodies in the manufacture of a medicament FOR TREATING ENDOTOXIN SHOCK**
Verwendung von Anti-ICAM Antikörpern zur Herstellung eines Präparats für die BEHANDLUNG VON ENDOTOXINSCHOCK
Utilisation d'anticorps anti-ICAM dans la fabrication d'un médicament destiné au traitement dES CHOCS ENDOTOXIQUES

(30) Priority: 31.08.1990 US 579137
(43) Date of publication of application: 16.06.1993
(73) Proprietor: BOEHRINGER INGELHEIM PHARMACEUTICALS INC., Ridgefield, Connecticut 06877-0368 (US)
(72) Inventor: LETTS, Lindsay, Gordon, Newtown, CT 06478 (US); WELDON, Steven, M., New Fairfield, CT 06812 (US); POSSANZA, Genus, J., Ridgefield, CT 06877 (US)
(74) Representative: Laudien, Dieter, Dr.
(86) International application number: US9106219
(87) International publication number: WO9204034

(56) References cited:
- EP-A- 0 314 863
- EP-A- 0 346 078
- THE FASEB JOURNAL vol. 5, no. 4, 11 March 1991, page A512 R. WINN ET AL. 'THE ROLE OF INTERCELLULAR ADHESION MOLECULE-1 (ICAM-1) IN ORGAN INJURY AFTER HEMORRHAGIC SHOCK IN PRIMATES'
- CHEMICAL ABSTRACTS, vol. 113, no. 21, 19 November 1990, Columbus, Ohio, US; abstract no. 184709u, ROCKEFELLER UNIVERSITY 'ANTI-CD-18 ANTIBODY FOR TREATING INFLAMMATION INDUCED BY INVADING LEUCOCYTES IN LUNG OR OTHER ORGANS' page 67 ;column 2 ;
- THE FASEB JOURNAL vol. 3, no. 3, 1989, page A447 C.M. DOERSCHUK ET AL. 'ROLE OF CD11/CD18 IN NEUTROPHIL (PMN) EMIGRATION IN RESPONSE TO ENDOTOXIN-INDUCED PNEUMONIA IN RABBITS'
- J. EXP. MED. vol. 170, no. 3, 1989, pages 959 - 969 E.I. TUOMANEN ET AL. 'REDUCTION OF INFLAMMATION, TISSUE DAMAGE, AND MORTALITY IN BACTERIAL MENINGITIS IN RABBITS TREATED WITH MONOCLONAL ANTIBODIES AGAINST ADHESION- PROMOTING RECEPTORS OF LEUKOCYTES'
- Journal of Immunology, Vol. 144(7), issued 1990, see Biological Abstract, No. 90: 23654, TANCHBURY et al., "A Monoclonal antibody that detects a novel antigen that is induced by Tumor Necrosis Factor IL-1 or lipopolysaccharide".
- J. of Surg. Res. (1990), vol.49, p.186-95
- The Merck Manual (1987), 15th Ed., Merck Sharp and Dohme Res. Ed., p.64-66

## Description

This invention relates a novel use of an anti-ICAM-1 antibody or fragment thereof in the preparation of an agent for treating endotoxin shock.

### BACKGROUND OF THE INVENTION

Cellular adhesion is a process through which leukocytes attach to cellular substrates, such as endothelial cells, in order to migrate from circulation to sites of ongoing inflammation, and properly defend the host against foreign invaders such as bacteria or viruses. An excellent review of the defense system is provided by Eisen, H.W., Microbiology, 3rd Ed., Harper & Row, Philadelphia, PA (1980), pp. 290-295 and 381-418.

One of the molecules on the surface of endothelial cells which participates in the adhesion process is the intercellular adhesion molecule ICAM-1. See Rothlein et al, J. Immunol. 137: 1270 (1986), herein incorporated by reference. This molecule has been shown to mediate adhesion by binding to molecules of the CD18 family of glycoproteins which are present on the cell surfaces of leukocytes [Sanchez-Madrid et al, J. Exper. Med. 158: 1785 (1983); Keiser et al, Eur. J. Immunol. 15: 1142 (1985)]. This glyprotein family is composed of heterodimers having one alpha chain and one beta chain. Although the alpha chain of each of the proteins differes from one another, the beta chain was found to be highly conserved (Sanchez-Madrid et al, supra). The beta chain of the glycoprotein family (sometimes referred to as "CD18") was found to have a molecular weight of 95 kd whereas the alpha chains were found to vary from 150 kd to 180 kd [Springer, T., Fed. Proc. 44: 2660 (1985)]. Although the alpha subunits of the membrane proteins do not share the extensive homology shared by the beta subunits, close analysis of the alpha subunits of the glycoproteins has revealed that there are substantial similarites between them. There are three major members of the CD18 family: Mac-1, LFA-1 and p150,95. Mac-1 is a heterodimer found on macrophages, granulocytes and large granular lymphocytes. LFA-1 is a heterodimer found on most lymphocytes [Springer et al, Immunol. Rev. 68: 111 (1982)]. p150,95 has a tissue distribution similar to Mac-1, and also plays a role in cellular adhesion [Keiser et al, Eur. J. Immunol. 15: 1142 (1985)]. Reviews of the similarities between the alpha and beta subunits of the LFA-1-related glycoproteins are provided by Sanchez-Madrid et al (supra) and Sanchez-Madrid et al, J. Exper. Med. 158: 586 (1983).

Anti-CD18 antibodies have been described as useful in treating hemorrhagic shock in rabbits. Vedder et al, J. Clin. Invest. 81: 939 (1988). Anti-CD18 antibodies have been shown not to increase susceptibility to sepsis when used to inhibit neutrophil adherence in rabbits. Mileski et al, Surgical Forum, Infection and its Mediators, p. 107 (1989).

U.S. Patent no. 4,388,318 describes a method for treating endotoxin shock using a pyrimido-pyrimidine derivative. U.S. Patents Nos. 4,472,420 and 4,472,431 describe a method for treating shock using benoxaprofen and fenoprofen, respectively.

European Patent Application 0 314 863, published on May 10, 1989, describes the use of anti-ICAM 1 antibodies or anti-CD18 antibodies for treating multiple organ injury secondary to septicemia.

European Patent Application 0 346 078, published on December 13, 1989, describes a method of inhibiting the influx of leukocytes and particular polymorphonuclear (PMN) leukocytes and of treating inflammation resulting from endotoxic shock using an anti-CD18 antibody.

Prior to present invention, however, no method was known for treating endotoxin shock using anti-ICAM-1 antibodies.

Accordingly, it is the purpose of this invention to provide a novel use of an anti-ICAM-1 antibody or fragment thereof in the preparation of an agent for treating endotoxin shock.

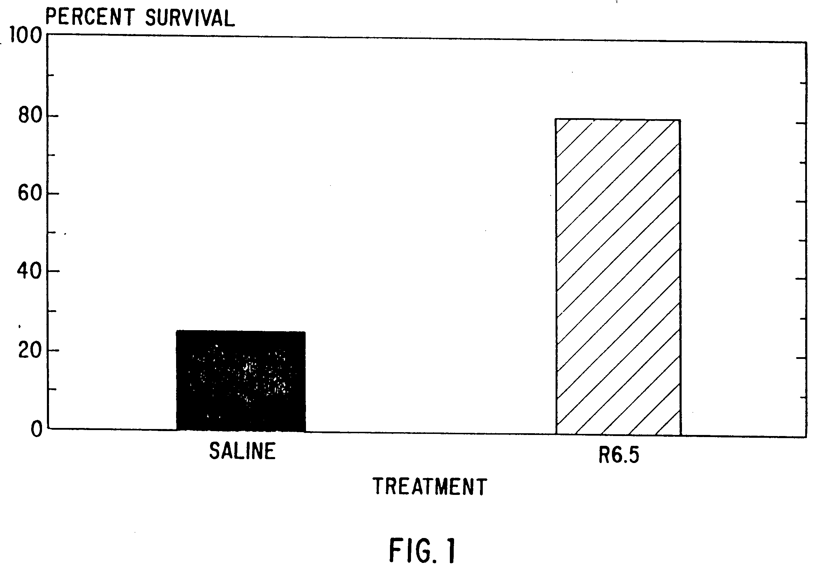

### BRIEF DESCRIPTION OF FIGURE 1

Figure 1 is a graphic representation of the survival of rabbits suffering from septic (endotoxin) shock, treated with saline or monoclonal anti-ICAM-1 antibody R6.5.

### DESCRIPTION OF THE INVENTION

This invention relates to the use of an anti-ICAM-1 antibody or fragment thereof in the preparation of an agent for treating endotoxin shock.

The anti-ICAM-1 antibody useful in this invention, can be prepared using known procedures, e.g., by immunopurification of polyclonal serum, by hybridoma technology or by recombinant DNA techniques. See, e.g., Kohler et al, PNAS USA 77(4): 2197 (1980) and U.S. Patent No. 4,816,397.

Preferably, the anti-ICAM-1 antibody is a monoclonal antibody. Monoclonal anti-ICAM-1 antibody can be prepared in the following manner. Mice are immunized with ICAM-1 or ICAM-1 containing cells. The mice are later sacrificed and their spleens removed. Spleen cells are fused with myeloma cells to produce hybridomas. The hybridomas are cultivated and their supernatants screened for anti-ICAM-1 activity, e.g., by ELISA or RIA, or by an aggregation assay as described in Rothlein et al (supra). Hybridomas secreting monoclonal antibodies with desired anti-ICAM-1 activity are cloned and cultivated.

Several methods for administering the anti-ICAM-1 antibody to the patient can be used including, for example, intradermal, subcutaneous, intramuscular, intraperitoneal, and intravenous routes. Preferably, administration of the anti-ICAM-1 antibody utilizes subcutaneous or intramuscular injection of the anti-ICAM-1 antibody in the presence of various adjuvants. The amount of anti-ICAM-1 antibody to be admimistered will vary depending on the severity of the shock, route of administration, etc., but, in general, 0.1 mg/kg to 10 mg/kg or higher, of antibody can be administered to the patient. The anti-ICAM-1 antibody can be formulated with a suitable adjuvant, including, e.g., mineral gels such as aluminum hydroxide; surface active substances such lysolecithin; pluronic polyols; polyanions; peptides; or oil emulsions.

The anti-ICAM-1 antibodies can be utilized as whole antibodies in the method of this invention or as fragments. The anti-ICAM-1 antibody fragments must comprise at least the binding site of the anti-ICAM-1 antibody. Useful fragments of the anti-ICAM-1 antibody include the Fab and F(ab)₂ fragments. Fragments of the anti-ICAM-1 antibody can be prepared using known procedures, e.g., by digestion of the antibody with proteolytic enzymes or by recombinant DNA techniques.

The following example illustrates the present invention.

### EXAMPLE

### A. PREPARATION OF MONOCLONAL ANTI-ICAM-1 ANTIBODY (R6.5)

The monoclonal anti-ICAM-1 antibody R6.5 was prepared as described in European Patent Application No. 289,949, from hybridoma cell line R6'506'E9'B2 (ATCC HB 9580).

### B. PREPARATION OF ANIMALS

Male white New Zealand rabbits (Hazelton Research, Denver, PA), weighing between 2.0 and 4.0 kg, were given an intravenous injection with one of the following:
i) Sterile saline (0.2 ml/kg); or
ii) R6.5 (2.0 mg/kg).
The R6.5 was in a solution of sterile saline. Following a period of 0.5 hours, the rabbits were primed with 20 µg/kg lipopolysaccharide (LPS) (Sigma E. Coli. LPS, serotype 0111:B4, lot 39F4030), by intravenous injection into the right marginal ear vein. After 18 hours the rabbits were anesthetized by intramuscular ketamine (50 mg/kg)/xylazine (6 mg/kg) followed by a constant xylazine (0.32 mg/kg/min) infusion. Systemic blood pressure was monitored via the right carotid artery by a P23 10 Gould/Statham pressure transducer. Rabbits initially receiving sterile saline were then given a second intravenous injection of sterile saline (0.2 ml/kg) into the right marginal ear vein. Animals receiving R6.5 as a priming dose were given a second intravenous injection of R6.5 (2.0 mg/kg) delivered in a solution of sterile saline. 0.5 hours later, the rabbits were given an additional intravenous injection of LPS (400 µg/kg) and monitored for 2 hours.

Blood samples of 1.0 ml were taken from the rabbits via the carotid artery catheter before the initial injection of sterile saline or R6.5; before the second injection of either sterile saline or R6.5; before the second (additional) injection of LPS; and 30 minutes, 1 hour and 2 hours after the second injection of LPS.

### C. RESULTS

Only 1 of the 4 saline-treated rabbits survived the entire protocol (Figure 1). All of the saline-treated rabbits developed an inflammatory response (conjunctival discharge, rhinorrhea) following the initial LPS injection. Two the these saline-treated rabbits died prior to the second LPS injection. The other 2 rabbits experienced severe hypotension with 1 expiring within 30 minutes after the second LPS injection.

All 5 rabbits treated with R6.5 survived until the second LPS injection. There was noticeably less of an inflammatory response following the initial LPS injection in the R6.5-treated rabbits. Four out of the 5 rabbits treated with R6.5 survived the entire protocol (Figure 1). One of the R6.5-treated rabbits expired 30 minutes following the second LPS injection. In the 4 R6.5-treated rabbits which survived, mean arterial pressure decreased only slightly (16±5 mmHg) by the end of the experiment.

## Claims

1. The use of an anti-ICAM-1 antibody or fragment thereof in the preparation of an agent for treating endotoxin shock.

2. The use as claimed in claim 1 which is the use of the entire anti-ICAM-1 antibody.

3. The use as claimed in claim 1 which is the use of a fragment of the anti-ICAM-1 antibody.

4. The use as claimed in claim 3 wherein the fragment is an Fab or F(ab)₂ fragment.

5. The use as claimed in any one of claims 1 to 4 wherein the agent is suitable for intravenous administration.

6. The use as claimed in any one of claims 1 to 5 wherein the anti-ICAM-1 antibody is R6.5.

## Patentansprüche

1. Verwendung eines Anti-ICAM-1-Antikörpers oder eines Fragments davon bei der Herstellung eines Mittels zur Behandlung eines Endotoxin-Schocks.

2. Verwendung nach Anspruch 1, bestehend in der Verwendung des gesamten Anti-ICAM-1-Antikörpers.

3. Verwendung nach Anspruch 1, bestehend in der Verwendung eines Fragments des Anti-ICAM-1-Antikörpers.

4. Verwendung nach Anspruch 3, bei welcher das Fragment ein Fab-Fragment oder F(ab)₂-Fragment ist.

5. Verwendung nach einem der Ansprüche 1 bis 4, bei welcher das Mittel sich zur intravenösen Verabreichung eignet.

6. Verwendung nach einem der Ansprüche 1 bis 5, bei welcher der Anti-ICAM-1-Antikörper R6.5 ist.

## Revendications

1. Utilisation d'un anticorps anti-ICAM-1 ou d'un fragment de celui-ci dans la préparation d'un agent pour traiter le choc endotoxinique.

2. Utilisation selon la revendication 1 qui est l'utilisation de l'anticorps anti-ICAM-1 entier.

3. Utilisation selon la revendication 1 qui est l'utilisation d'un fragment de l'anticorps anti-ICAM-1.

4. Utilisation selon la revendication 3 dans laquelle le fragment est un fragment Fab ou F(ab)₂.

5. Utilisation selon l'une quelconque des revendications 1 à 4 dans laquelle l'agent est approprié à l'administration intraveineuse.

6. Utilisation selon l'une quelconque des revendications 1 à 5 dans laquelle l'anticorps anti-ICAM-1 est R6.5.
